# EUROPEAN PATENT APPLICATION

(11) **EP 3 479 786 A1**
(43) Date of publication of application: **08.05.2019**
(21) Application number: 16907959.7
(22) Date of filing: 02.08.2016
(51) Int. Cl.: A61B 18/12

(54) **BIPOLAR HIGH-FREQUENCY ELECTRIC KNIFE**

(30) Priority: 04.07.2016 CN 201610533233
(71) Applicant: Micro-Tech (Nanjing) Co., Ltd., Nanjing, Jiangsu 210000 (CN)
(72) Inventor: TANG, Zhi, Nanjing Jiangsu 210000 (CN); FAN, Mingqiao, Nanjing Jiangsu 210000 (CN); XU, Huiwen, Nanjing Jiangsu 210000 (CN); SHA, Deqing, Nanjing Jiangsu 210000 (CN)
(74) Representative: Fioravanti, Corrado
(86) International application number: PCT/CN2016/092846
(87) International publication number: WO 2018/006455

(57) **Abstract**

Disclosed is a bipolar high-frequency electric knife, comprising a cutter portion (21), a main body part (22) and an operation portion (23). The cutter portion (21) comprises a first electrode portion (3), a second electrode portion (1) and an insulation member (11). The main body part (22) comprises a position-limiting part (5) and an insulation sheath (6). The operation portion (23) comprises a handle (7), a button (8), a socket (9), a first wire (10a) and a second wire (10b). The bipolar high-frequency electric knife can not only prevent the perforation of the basal layer of tissue caused by high-frequency electric shock during cutting, but can also ensure that the two electrodes are simultaneously in full contact with the tissue in any direction or at any angle, thereby guaranteeing the reliability of surgical cutting.

## Description

### Technical Field

The present disclosure relates to a medical bipolar high-frequency electrotome (electric knife), particularly a bipolar high-frequency electrotome with a smallest loop and an insulation protection function.

### Technical Background

Endoscopic submucosal dissection (ESD) refers to an endoscopic minimally invasive technology for submucosal dissection of a lesion of greater than 2 cm by using a high-frequency instrument. Compared with conventional surgical operations, on the basis of effecting a radical cure of tumors, ESD well retains physiological functions of gastrointestinal tract, significantly improves patients' postoperative quality of life, and currently has become a preferred treatment method for early cancers and precancerous lesions of gastrointestinal tract including esophagus (Gotoda T, Kondo H, Ono H, et al. A new endoscopic mucosal resection procedure using an Insulation-tipped electrosurgical knife for rectal flat lesions: report of two cases[J]. Gastrointest Endosco, 1999, 50:560-563).

For ESD, a high-frequency current is output by an external device to form a loop by a human body, and a high-density current is formed at a knife wire portion with a relatively small sectional area to realize cutting. Currently, high-frequency knives for endoscopic submucosal dissection may be classified into two types according to manners in which currents thereof form the loops. One is monopolar high-frequency electrotome, and the other is bipolar high-frequency electrotome. The monopolar high-frequency electrotome has a shortcoming that the high-frequency current will pass through most area of the human body. The high-frequency current of the bipolar high-frequency electrotome, however, merely flows through a minimum area of the human tissues, that is, merely flows around lesion tissues. Since the high-frequency current passes through a small area of the human body, and will not injure muscularis mucosae, the bipolar high-frequency electrotome is considered as a safest high-frequency electrotome. However, in order to ensure that the high-frequency current can successfully reach lesions tissues to be treated via a conducting wire, sufficient contact of an active electrode and an inert electrode with human tissue parts has to be ensured, but current product designs of the bipolar high-frequency electrotome can hardly ensure that the active electrode and the inert electrode are simultaneously in contact with the tissues during a surgical process, particularly at some parts of natural orifices of the human body where it is difficult to perform an operation, formation of a current loop can hardly be ensured, thus resulting in a unreliable and unstable cutting, which directly adversely affecting operation efficiency and patients' safety. Therefore, it is necessary to develop a bipolar high-frequency electrotome which is more reliable and stable in cutting, with insulation protection for a cutter head, and higher safety performance.

### Summary

An object of the present disclosure is to provide a bipolar high-frequency electrotome for cutting and dissection in treatment of gastrointestinal tract early cancers and precancerous lesions, which is provided with a minimum stable loop and an insulation protection head, to ensure reliability and safety of surgical cutting.

The present disclosure provides a bipolar high-frequency electrotome, comprising a cutter portion, a main body part, and an operation portion.

The cutter portion, provided at a distal end of the bipolar high-frequency electrotome, comprises a first electrode portion, a second electrode portion, and an insulation member; wherein the first electrode portion is an active electrode for tissue cutting, can be pushed out or taken back with respect to a front end of the main body part, and comprises a tubular portion and a bulge; the second electrode portion is an inert electrode, and comprises a rod-shape portion and a protruding portion which is located at a distal end of the rod-shape portion; the protruding portion of the second electrode portion is located at a distal end of the first electrode portion; a rod-shape portion of the second electrode portion is inserted into the first electrode portion; and the insulation member is configured to insulate the first electrode portion from the second electrode portion.

The main body part comprises an insulation sheath and a position-limiting part. The insulation sheath is insulative at least on an outer circumferential face, and runs between the cutter portion and the operation portion; the position-limiting part is located inside the insulation sheath, and comprises a fixed insulation part for position-limiting and a movable part for position-limiting.

The operation portion is provided at a proximal end side of the main body part, has a first conducting wire connected with the first electrode portion, and has a second conducting wire connected with the second electrode portion, and can enable the cutter portion to be pushed out or taken back with respect to the front end of the main body part.

Preferably, an extent to which the distal end of the first electrode portion extends outwards in a direction perpendicular to an axis of the main body part is greater than a radius of a cross section of the tubular portion of the first electrode portion, forming a bulge at the distal end of the first electrode portion.

Preferably, a cross section of the bulge is circular or polygonal. Preferably, an extent to which the protruding portion of the second electrode portion extends outwards in a direction perpendicular to an axis of the rod-shape portion of the second electrode portion is greater than a radius of the rod-shape portion of the second electrode portion, a surface of the protruding portion of the second electrode portion is a smooth surface without sharp edges, and the protruding portion of the second electrode portion is located at the distal end of the first electrode portion.

Preferably, the insulation member comprises an insulation part and an insulation sleeve. The insulation part is located between the first electrode portion and the protruding portion of the second electrode portion, for insulating the first electrode portion from the protruding portion of the second electrode portion; the insulation sleeve wraps an outer surface of the rod-shape portion of the second electrode portion, and the first electrode portion wraps an outer surface of the insulation sleeve, the rod-shape portion of the second electrode portion, the insulation sleeve and the first electrode portion form a concentric structure.

Preferably, the insulation part is embedded in the protruding portion of the second electrode portion or is flush with an end face of the protruding portion of the second electrode portion, and the first electrode portion is embedded in the insulation part.

Preferably, the position-limiting part comprises a fixed insulation part for position-limiting fixed at a distal end of the insulation sheath, and a movable part for position-limiting provided at a proximal end of the first electrode portion, an extent to which the movable part for position-limiting extends outwards in a direction perpendicular to the axis of the main body part is smaller than an extent to which the fixed insulation part for position-limiting extends outwards in the direction perpendicular to the axis of the main body part, and the fixed insulation part for position-limiting has a distal-end end face beyond or at least flush with a distal-end end face of the insulation sheath, and has a proximal-end end face inserted into the insulation sheath.

Preferably, an outer surface of the fixed insulation part for position-limiting is roughened or barbed.

Preferably, the operation portion further comprises a handle, a button is provided on the handle, and the button on the handle can linearly slide along an axis of the handle, realizing actions of push-out and take-back of the cutter portion by pushing forward or pulling backward the button.

The present disclosure further provides a bipolar cutter head of a high-frequency electrotome, comprising:
a first electrode portion, a second electrode portion, and an insulation member. The first electrode portion is an active electrode for tissue cutting, can be pushed out or taken back with respect to a front end of a main body part, and comprises a tubular portion and a bulge; the second electrode portion is an inert electrode, and comprises a rod-shape portion and a protruding portion which is located at a distal end of the rod-shape portion; the protruding portion of the second electrode portion is located at a distal end of the first electrode portion; the rod-shape portion of the second electrode portion is inserted into the first electrode portion, an extent to which the protruding portion of the second electrode portion extends outwards in a direction perpendicular to an axis of the rod-shape portion of the second electrode portion is greater than a radius of the rod-shape portion of the second electrode portion, and a surface of the protruding portion of the second electrode portion is a smooth surface without sharp edges; and the insulation member is configured to insulate the first electrode portion from the second electrode portion.

Preferably, an extent to which the distal end of the first electrode portion extends outwards in a direction perpendicular to an axis of the main body part is greater than a radius of a cross section of the tubular portion of the first electrode portion, forming a bulge at the distal end of the first electrode portion.

Preferably, a cross section of the bulge is circular or polygonal.

Preferably, the insulation member comprises an insulation part and an insulation sleeve. The insulation part is located between the first electrode portion and the protruding portion of the second electrode portion, for insulating the first electrode portion from the protruding portion of the second electrode portion; the insulation sleeve wraps an outer surface of the rod-shape portion of the second electrode portion, and the first electrode portion wraps an outer surface of the insulation sleeve, the rod-shape portion of the second electrode portion, the insulation sleeve and the first electrode portion form a concentric structure.

Preferably, the protruding portion of the second electrode portion is located at the distal end of the first electrode portion.

Preferably, the insulation part is embedded in the protruding portion of the second electrode portion or is flush with an end face of the protruding portion of the second electrode portion, and the first electrode portion is embedded in the insulation part.

### Beneficial Effects:

With respect to the existing bipolar electrotomes, the bipolar high-frequency electrotome provided in the present disclosure has a smaller current loop with two electrodes, and has insulation protection for a head end, thus enabling a safer operation.

With respect to the existing bipolar electrotomes, for the bipolar high-frequency electrotome provided in the present disclosure, with the inert electrode provided in front, and the active electrode provided behind, when the electrotome gets into, under guidance of an endoscope, a gastrointestinal lesion from a natural orifice (an oral cavity, an anal cavity) of the human body, the both electrodes can be ensured to contact the tissues at any angle or in any direction, so as to form a current loop for tissue cutting, ensuring reliability of the surgical cutting, and enabling the operation to be safer.

For the bipolar high-frequency electrotome provided in the present disclosure, an insulation protection layer is provided between the active electrode and the inert electrode, which can prevent perforation of a basal layer of tissues caused by high-frequency electric shock during cutting.

For the bipolar high-frequency electrotome provided in the present disclosure, a section area of the inert electrode is greater than a section area of the active electrode, and providing the inert electrode is at the distal end, can serve a protective function of preventing perforation and bleeding during cutting.

For the bipolar high-frequency electrotome provided in the present disclosure, during cutting the bulge formed at a portion of the distal end of the active electrode in contact with the insulation part can lift up tissues swelling up after liquid injection, such that the tissues are away from muscularis mucosae to prevent perforation, meanwhile, the bulge can cut the tissues when the cutter portion moves longitudinally.

### Brief Description of Drawings

FIG. 1 is an overall structural diagram of a bipolar high-frequency electrotome;
FIG. 2 is a partial enlarged view of a cutter head of the bipolar high-frequency electrotome;
FIG. 3A-FIG. 3B are cutaway views of electrodes and insulation part in combination of the bipolar high-frequency electrotome;
FIG. 4A-FIG. 4D are views of the electrotome at different cutting angles;
FIG. 5A-FIG. 5D illustrate a process of using the electrotome;
FIG. 6A-FIG. 6B illustrate a cutting process of the electrotome;
FIG. 7A-FIG. 7B are cross-sectional cutaway views of a bulge of a first electrode portion.

1. second electrode portion, 1a. rod-shape portion, 1b. protruding portion, 2. insulation part, 3. first electrode portion, 4. insulation sleeve, 5. position-limiting part, 6. insulation sheath, 7. handle, 8. button, 9. socket, 10a. first conducting wire, 10b. second conducting wire, 11. insulation member, 12. fixed insulation part for position-limiting, 13. movable part for position-limiting, 16. lesion tissue, 17. muscularis mucosae, 18. tubular portion, 19. bulge, 21. cutter portion, 22. main body part, 23. operation portion, 31. first electrode, 32. second electrode, 33. insulation portion

### Detailed Description of Embodiments

In order to make the object, technical solutions, and advantages of the present disclosure more clear and understandable, the present disclosure is further described in detail below in combination with accompanying drawings and embodiments. It should be understood that the embodiments described herein are merely used to explain the present disclosure rather than limiting the present disclosure.

The present disclosure is further described below in combination with the accompanying drawings and the embodiments, but the present disclosure absolutely is not limited to the following embodiments.

### Embodiment 1

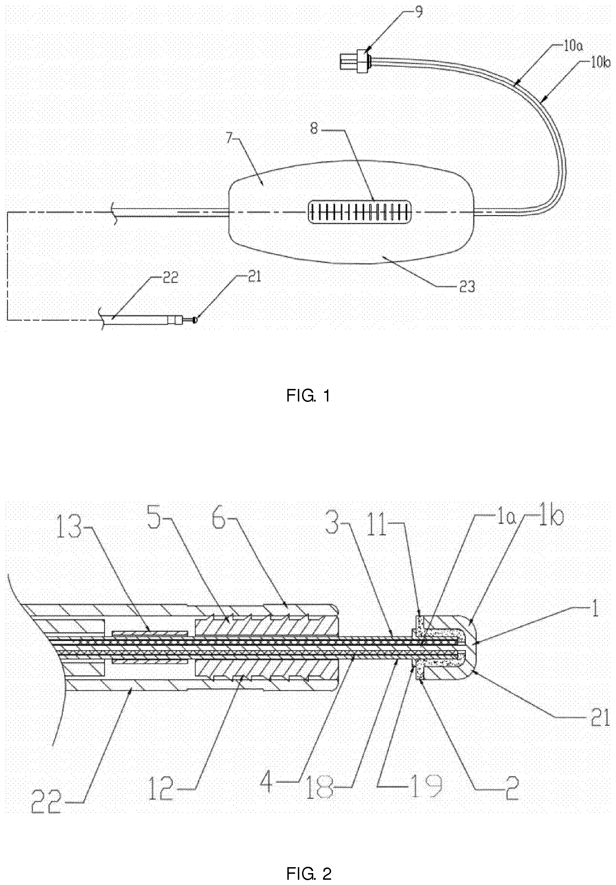

As shown in FIG. 1, a bipolar high-frequency electrotome in the present embodiment comprises a cutter portion 21, a main body part 22, and an operation portion 23.

Hereinafter, an end of the cutter portion 21 is referred to as a distal end, and an end of the operation portion 23 is referred to as a proximal end.

As shown in FIG. 2, the cutter portion 21 is provided at the distal end of the bipolar high-frequency electrotome, and comprises a first electrode portion 3, a second electrode portion 1, and an insulation member 11. The cutter portion 21 can move in an axial direction of the main body part 22, and can project out and get in with respect to a front end of the main body part 22.

The first electrode portion 3 is an active electrode for tissue cutting, and can be pushed out or taken back with respect to the front end of the main body part 22. The first electrode portion comprises a tubular portion 18 and a bulge 19. The second electrode portion 1 is an inert electrode, and comprises a rod-shape portion 1 a and a protruding portion 1b which is located at a distal end of the rod-shape portion 1a. The protruding portion 1b of the second electrode is located at a distal end of the first electrode portion 3, and an extent to which the protruding portion 1b of the second electrode portion 1 extends outwards in a direction perpendicular to an axis of the rod-shape portion 1a of the second electrode portion 1 is greater than a radius of the rod-shape portion 1a of the second electrode portion 1. A surface of the protruding portion 1b of the second electrode portion 1 is a smooth surface without sharp edges. A cross section diameter of the protruding portion is greater than a cross section diameter of the first electrode portion 3. The rod-shape portion 1a of the second electrode portion is inserted into the first electrode portion 3 and is in communication with the operation portion 23. The insulation member 11 is configured to insulate the first electrode portion 3 from the second electrode portion 1, ensuring that the active electrode and the inert electrode are simultaneously in contact with tissues during a surgical process, and the insulation member comprises an insulation part 2 and an insulation sleeve 4. The insulation part 2 is located between the first electrode portion 3 and the protruding portion 1b of the second electrode portion 1, for insulating the first electrode portion 3 from the protruding portion 1b of the second electrode portion 1. The insulation sleeve 4 wraps an outer surface of the rod-shape portion of the second electrode portion, and the first electrode portion wraps an outer surface of the insulation sleeve 4, the rod-shape portion of the second electrode portion, the insulation sleeve and the first electrode portion form a concentric structure.

The insulation member 11 is of an insulation material, and is preferably made of a thermal resistant material such as a ceramic material.

Insulating the first electrode portion from the second electrode portion using the insulation material prevents failure of the electrotome after the first electrode and the second electrode are in direct conductive connection. With the inactive second electrode portion provided in front, and the active first electrode portion provided behind, when the electrotome gets into, under guidance of an endoscope, a gastrointestinal lesion from a natural orifice (an oral cavity, an anal cavity) of the human body, the both electrode portions can be ensured to contact the tissues at any angle or in any direction, so as to form a current loop for tissue cutting, ensuring reliability of the surgical cutting, and enabling a safer operation.

The main body part 22 comprises an insulation sheath 6 and a position-limiting part 5. The insulation sheath has an outer diameter and flexibility enabling insertion of the insulation sheath through a channel of the endoscope, and is insulative at least on an outer circumferential face and can be resistant to high temperature, and the insulation sheath runs between the cutter portion and the operation portion. The position-limiting part 5 is located inside the insulation sheath 6, for defining a push-out amount of the first electrode portion being pushed out from a distal end of the insulation sheath; the position-limiting part functions of positionally limiting and protecting the cutter portion 21 when taken back into the insulation sheath. When the cutter portion is pushed out, the first electrode portion 3 performs cutting, and when the cutter portion is taken back, the cutter head returns back into the insulation sheath 6, to be positionally limited and protected by the position-limiting part 5.

The position-limiting part 5 comprises a fixed insulation part for position-limiting 12 fixed at the distal end of the insulation sheath, and a movable part for position-limiting 13 provided at a proximal end of the first electrode portion 3. The movable part for position-limiting 13 and the fixed insulation part for position-limiting 12 perform the position limiting by means of steps formed due to different sizes, so as to control an extension amount of the first electrode portion.

The position-limiting part 5 comprises the fixed insulation part for position-limiting 12 fixed at the distal end of the insulation sheath, and the movable part for position-limiting 13 provided at the proximal end of the first electrode portion. An extent to which the movable part for position-limiting 13 extends outwards in a direction perpendicular to the axis of the main body part is smaller than an extent to which the fixed insulation part for position-limiting 12 extends outwards in a direction perpendicular to the axis of the main body part. The fixed insulation part for position-limiting 12 has a distal-end end face beyond or at least flush with a distal-end end face of the insulation sheath, and a proximal-end end face inserted into the insulation sheath. The movable part for position-limiting 13 is a metal part, and is fixed to a fixed point of the first electrode portion 3. The fixed insulation part for position-limiting 12 is fixed at the distal end of the insulation sheath, and the fixed insulation part for position-limiting 12 is made of an insulation material resistant to high temperature, preferably made of a thermal resistant material such as the ceramic material. A surface of the fixed insulation part for position-limiting 12 is roughened or barbed to increase a frictional force with the insulation sheath 6.

When a button 8 on the operation portion 23 is pushed, the first electrode portion 3 and the movable part for position-limiting 13 are driven to extend together towards the distal end. When the distal-end end face of the movable part for position-limiting 13 comes into contact with the proximal-end end face of the fixed insulation part for position-limiting 12, the movable part for position-limiting cannot continue to extend towards the distal end due to a size difference therebetween, thus a position-limiting function is achieved. When the button 8 on the operation portion 23 is pulled back, the cutter portion 21 is driven to get back into the insulation sheath 6. When the insulation part 2 in the cutter portion 21 comes into contact with a side face of the fixed insulation part for position-limiting 12, the steps formed due to the size difference prevent the cutter portion 21 from continuing to return, thus a position-limiting function is effected, meanwhile, the fixed insulation part for position-limiting 12 is of an insulation material, which well protects insulativity of other parts of the whole electrotome except the active electrode.

As shown in FIG. 1, the operation portion 23 is provided at a proximal end side of the main body part 22, and can enable the cutter portion 21 to be pushed out or taken back with respect to the front end of the main body part 22. The operation portion comprises a handle 7, the button 8, a socket 9, a first conducting wire 10a connected with the first electrode portion 3, and a second conducting wire 10b connected with the second electrode portion 1. The first electrode portion 3 and the second electrode portion 1 are connected to a tail portion of the handle via the conducting wires respectively, and are respectively guided by the first conducting wire 10a and the second conducting wire 10b onto the socket 9, and the two portions are connected to a high-frequency generator via the socket 9 to input a high-frequency current to the electrotome. The button 8 on the handle 7 can linearly slide along an axis of the handle 7, realizing actions of push-out and take-back of the cutter portion by pushing forward or pulling backward the button 8.

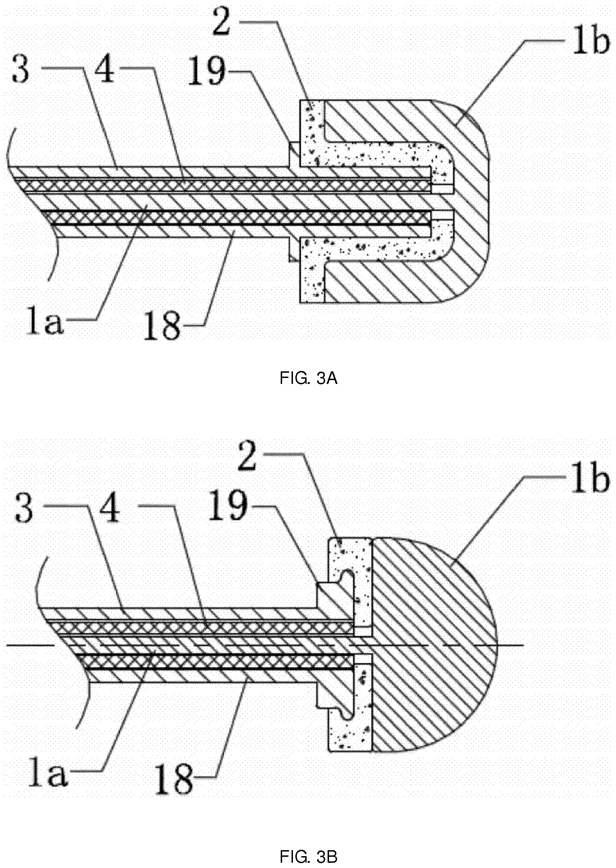

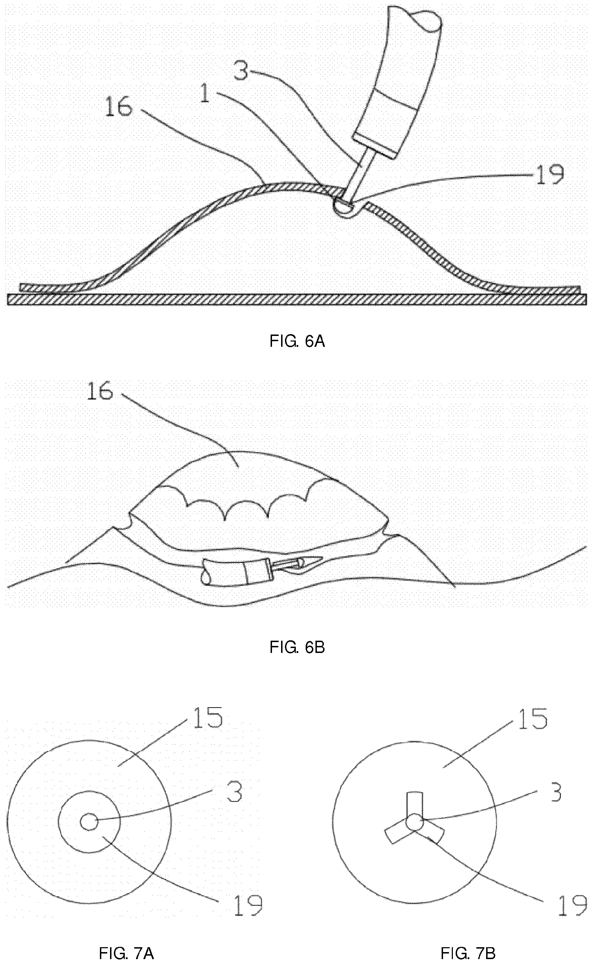

As shown in FIG. 3A-FIG. 3B, the insulation member 11 shown comprises the insulation part 2 and the insulation sleeve 4. The insulation part 2 is located between the first electrode portion 3 and the protruding portion 1b of the second electrode portion 1, for insulating the first electrode portion 3 from the protruding portion 1b of the second electrode portion 1, such that the active electrode and the insert electrode can be effectively insulated, ensuring insulation between the two electrodes. The insulation sleeve 4 wraps an outer surface of the rod-shape portion of the second electrode portion, and the first electrode portion wraps an outer surface of the insulation sleeve 4, the rod-shape portion of the second electrode portion, the insulation sleeve and the first electrode portion form a concentric structure. The insulation part 2 is embedded in the protruding portion 1b of the second electrode portion or is flush with an end face of the protruding portion 1b of the second electrode portion, and the first electrode portion 3 is embedded in the insulation part 2. A portion of the first electrode portion 3 parallel to the insulation sleeve 4 is the tubular portion 18 of the first electrode portion. An extent to which the distal end of the first electrode portion 3 extends outwards in a direction perpendicular to the axis of the main body part, is greater than a radius of a cross section of the tubular portion 18 of the first electrode portion 3, forming a bulge 19 at the distal end of the first electrode portion 3. A cross section of the bulge 19 may be circular or polygonal, or in other shapes, as shown in FIG. 7A and FIG. 7B. During cutting, the bulge 19 can lift up tissues swelling up after liquid injection, such that the tissues are away from muscularis mucosae to prevent perforation, meanwhile, the bulge 19 can cut the tissues when the cutter portion 21 moves longitudinally.

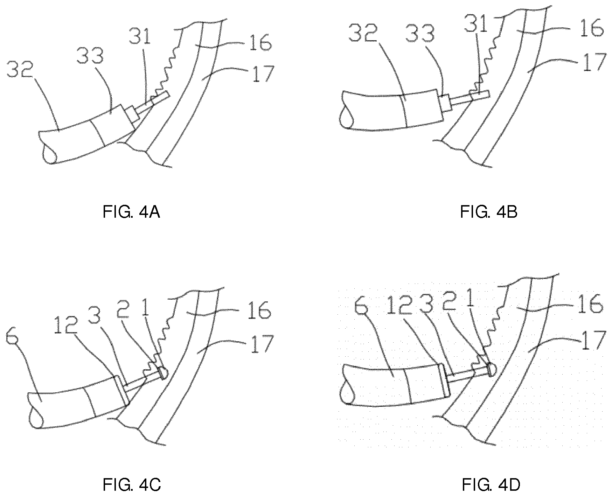

FIG. 4A-FIG. 4D are views of the electrotome at different cutting angles. FIG. 4A and FIG. 4B represents examples of different cutting angles of a conventional bipolar electrotome in the prior art. FIG. 4C and FIG. 4D show the bipolar high-frequency electrotome provided in the present disclosure. As shown in FIG. 4A and FIG. 4B, in the prior art, a first electrode 31 of the bipolar electrotome is located at a distal end of a second electrode 32, and an insulation portion 33 is provided between the first electrode 31 and the second electrode 32, then only at certain specific angles (as shown in FIG. 4A) can the requirement that the two electrodes are simultaneously in contact with the tissues be met, thus achieving an object of tissue cutting. When at the cutting angles as shown in FIG. 4A and FIG. 4C, the first electrode and the second electrode simultaneously contact a mucosal layer of lesion 16 without contacting the muscularis mucosae 17, and when at the cutting angles as shown in FIG. 4B and FIG. 4D, for the conventional bipolar electrotome, when it is ensured that the first electrode does not contact the muscularis mucosae 17, the second electrode no longer contacts the lesion mucosa 16, leading to an interruption of the loop, thus the cutting can no longer continue, and in order to ensure the proceeding of the cutting, the first electrode has to be inserted deep into the muscularis mucosae 17, thus increasing the possibility of bleeding and perforation. For the bipolar high-frequency electrotome provided in the present disclosure, since the protruding portion 1b of the second electrode portion 1 is located at the distal end of the first electrode portion 3, at the same cutting angle (as shown in FIG. 4D), the first electrode portion 3 and the second electrode portion 1 still can be ensured to simultaneously contact the lesion tissue 16, and the two electrodes can be ensured to contact the tissues at any angle or in any direction. Moreover, the protruding portion 1b of the second electrode portion 1 is an insert electrode, then even if it is inserted too deep into the muscularis mucosae 17 in operation, no cutting will occur, functioning to prevent bleeding and perforation, and achieving better technical effects.

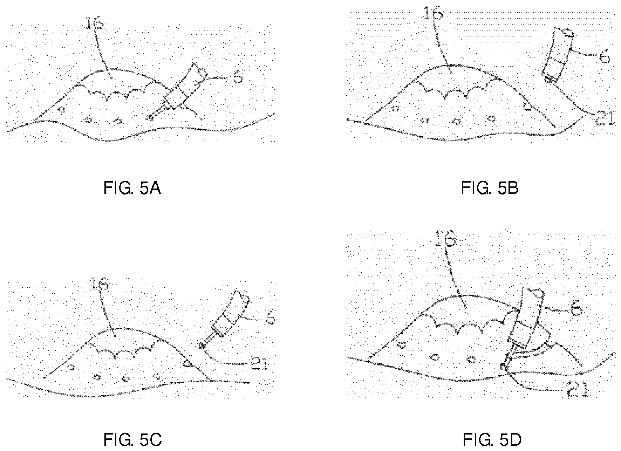

An operation process for the bipolar high-frequency electrotome is illustrated with FIG. 5A-FIG. 5D:
In a surgical process, a surgeon firstly marks around the lesion tissues 16 by a needle-shape knife (as shown in FIG. 5A), then he injects normal saline into the lesion tissues 16 using an injection needle to make the lesion tissues swell up, next he inserts the main body part 22 of the high-frequency electrotome to the vicinity of the lesion tissues 16 through a channel of an endoscope. In this process, the cutter portion 21 should be kept to be stored inside the sheath 7 (as shown in FIG. 5B), to protect the cutter portion 21 and the endoscope against damage in this process. After arriving at the lesion tissues 16, the cutter portion 21 is pushed out by the button 8 of the operation portion 23 (as shown in FIG. 5C), then the cutter portion is placed in an initial incision formed by the needle-shape knife, then on one hand, the first electrode portion 3 is supplied with a high-frequency current, and on the other hand, the cutter performs cutting along a direction in FIG. 5D (as shown in FIG. 5D). For places of the gastrointestinal tract where it is inconvenient to carry out some transverse cutting operations, longitudinal cutting may be used instead, as shown in FIG. 6A-FIG. 6B. After the tissues are lifted up, the lesion tissues 16 gather at the bulge 19 of the first electrode portion 3, at which time the bulge 19 has a function of cutting.

The above-mentioned are merely preferred embodiments of the present disclosure, such that a person skilled in the art can understand or implement the invention of the present disclosure. Various modifications and combinations to these embodiments are apparent to a person skilled in the art, and general principles defined herein can be implemented in other embodiments without departing from the spirit or scope of the present disclosure. Therefore, the present disclosure will not be limited to these embodiments shown in the text, but should conform to the broadest scope consistent to the principle and novel features disclosed herein.

## Claims

1. A bipolar high-frequency electrotome, **characterized by** comprising:
a cutter portion, a main body part, and an operation portion,
wherein the cutter portion is provided at a distal end of the bipolar high-frequency electrotome and comprises a first electrode portion, a second electrode portion, and an insulation member; the first electrode portion is an active electrode for tissue cutting, is capable of being pushed out or taken back with respect to a front end of the main body part, and comprises a tubular portion and a bulge; the second electrode portion is an inert electrode, and comprises a rod-shape portion and a protruding portion which is located at a distal end of the rod-shape portion; the protruding portion of the second electrode is located at a distal end of the first electrode portion; the rod-shape portion of the second electrode portion is inserted into the first electrode portion; the insulation member is configured to insulate the first electrode portion from the second electrode portion;
the main body part comprises an insulation sheath and a position-limiting part, the insulation sheath is insulative at least on an outer circumferential face, and runs between the cutter portion and the operation portion; the position-limiting part is located inside the insulation sheath and comprises a fixed insulation part for position-limiting and a movable part for position-limiting; and
the operation portion is provided at a proximal end side of the main body part and has a first conducting wire connected with the first electrode portion, and has a second conducting wire connected with the second electrode portion, and the operation portion is capable of enabling the cutter portion to be pushed out or taken back with respect to the front end of the main body part.

2. The bipolar high-frequency electrotome according to claim 1, wherein an extent to which the distal end of the first electrode portion extends outwards in a direction perpendicular to an axis of the main body part is greater than a radius of a cross section of the tubular portion of the first electrode portion, forming a bulge at the distal end of the first electrode portion.

3. The bipolar high-frequency electrotome according to claim 2, wherein a cross section of the bulge is circular or polygonal.

4. The bipolar high-frequency electrotome according to claim 1, wherein an extent to which the protruding portion of the second electrode portion extends outwards in a direction perpendicular to an axis of the rod-shape portion of the second electrode portion is greater than a radius of the rod-shape portion of the second electrode portion, a surface of the protruding portion of the second electrode portion is a smooth surface without sharp edges, and the protruding portion of the second electrode portion is located at the distal end of the first electrode portion.

5. The bipolar high-frequency electrotome according to claim 1, wherein the insulation member comprises an insulation part and an insulation sleeve, the insulation part is located between the first electrode portion and the protruding portion of the second electrode portion, for insulating the first electrode portion from the protruding portion of the second electrode portion; the insulation sleeve wraps an outer surface of the rod-shape portion of the second electrode portion, the first electrode portion wraps an outer surface of the insulation sleeve, and the rod-shape portion of the second electrode portion, the insulation sleeve and the first electrode portion form a concentric structure.

6. The bipolar high-frequency electrotome according to claim 5, wherein the insulation part is embedded in the protruding portion of the second electrode portion or is flush with an end face of the protruding portion of the second electrode portion, and the first electrode portion is embedded in the insulation part.

7. The bipolar high-frequency electrotome according to claim 1 or 2, wherein the position-limiting part comprises the fixed insulation part for position-limiting which is fixed at a distal end of the insulation sheath, and the movable part for position-limiting which is provided at a proximal end of the first electrode portion, an extent to which the movable part for position-limiting extends outwards in a direction perpendicular to an axis of the main body part is smaller than an extent to which the fixed insulation part for position-limiting extends outwards in the direction perpendicular to the axis of the main body part, and the fixed insulation part for position-limiting has a distal-end end face beyond or at least flush with a distal-end end face of the insulation sheath, and a proximal-end end face inserted into the insulation sheath.

8. The bipolar high-frequency electrotome according to claim 6, wherein an outer surface of the fixed insulation part for position-limiting is roughened or barbed.

9. The bipolar high-frequency electrotome according to claim 1, wherein the operation portion further comprises a handle, a button is provided on the handle, the button on the handle is capable of linearly sliding along an axis of the handle, realizing actions of pushing-out and taking-back the cutter portion by pushing forward or pulling backward the button.

10. A bipolar cutter head of a high-frequency electrotome, **characterized by** comprising:
a first electrode portion, a second electrode portion, and an insulation member,
wherein the first electrode portion is an active electrode for tissue cutting, is capable of being pushed out or taken back with respect to a front end of a main body part, and comprises a tubular portion and a bulge;
the second electrode portion is an inert electrode, and comprises a rod-shape portion and a protruding portion which is located at a distal end of the rod-shape portion; the protruding portion of the second electrode is located at a distal end of the first electrode portion; the rod-shape portion of the second electrode portion is inserted into the first electrode portion, an extent to which the protruding portion of the second electrode portion extends outwards in a direction perpendicular to an axis of the rod-shape portion of the second electrode portion is greater than a radius of the rod-shape portion of the second electrode portion, and a surface of the protruding portion of the second electrode portion is a smooth surface without sharp edges; and
the insulation member is configured to insulate the first electrode portion from the second electrode portion.

11. The bipolar cutter head of a high-frequency electrotome according to claim 10, wherein an extent to which the distal end of the first electrode portion extends outwards in a direction perpendicular to an axis of the main body part is greater than a radius of a cross section of the tubular portion of the first electrode portion, forming a bulge at the distal end of the first electrode portion.

12. The bipolar cutter head of a high-frequency electrotome according to claim 11, wherein a cross section of the bulge is circular or polygonal.

13. The bipolar cutter head of a high-frequency electrotome according to claim 10, wherein the insulation member comprises an insulation part and an insulation sleeve, the insulation part is located between the first electrode portion and the protruding portion of the second electrode portion, for insulating the first electrode portion from the protruding portion of the second electrode portion; the insulation sleeve wraps an outer surface of the rod-shape portion of the second electrode portion, the first electrode portion wraps an outer surface of the insulation sleeve, and the rod-shape portion of the second electrode portion, the insulation sleeve and the first electrode portion form a concentric structure.

14. The bipolar cutter head of a high-frequency electrotome according to claim 13, wherein the insulation part is embedded in the protruding portion of the second electrode portion or is flush with an end face of the protruding portion of the second electrode portion, and the first electrode portion is embedded in the insulation part.
